# EUROPEAN PATENT SPECIFICATION

(11) **EP 1 501 503 B1**
(45) Date of publication and mention of the grant of the patent: **24.12.2008**
(21) Application number: 03727938.7
(22) Date of filing: 07.05.2003
(51) Int. Cl.: A61K 31/405, C07D 209/18

(54) **SUBSTANTIALLY PURE SOLID FORM OF THE ENOL TAUTOMER OF 3-INDOLYPYRUVIC ACID FOR USE IN THE TREATMENT OF CENTRAL NERVOUS SYSTEM DISTURBANCES**
ENOL-TAUTOMER DER 3-INDOL-BRENZTRAUBENSÄURE IN FESTER UND IM WESENTLICHEN REINER FORM ZUR BEHANDLUNG VON ERKRANKUNGEN DES ZENTRALEN NERVENSYSTEMS
FORME SOLIDE ESSENTIELLEMENT PURE DU TAUTOMERE ENOLIQUE DE L'ACIDE 3-INDOLYLPYRUVIQUE, UTILISE DANS LE TRAITEMENT DES TROUBLES DU SYSTEME NERVEUX CENTRAL

(30) Priority: 09.05.2002 IT RM20020255
(43) Date of publication of application: 02.02.2005
(73) Proprietor: POLIFARMA S.p.A., 00144 Roma RM (IT)
(72) Inventor: POLITI, Vincenzo, I-00179 Roma (IT); SELLA, Antonio, I-00171 Roma (IT); BARTOLINI, Barbara, I-00144 Roma RM (IT); MARGONELLI, Andrea, I-00147 Roma (IT); SOMMA, Francesca, I-00133 Roma (IT); CORNIELLO, Cristina, I-00135 Roma (IT)
(74) Representative: Germinario, Claudio
(86) International application number: PCT/IT2003/000275
(87) International publication number: WO 2003/094914

(56) References cited:
- EP-A- 0 421 946
- GB-A- 1 128 607
- JP-B- 34 004 274
- US-A- 4 005 206
- DATABASE CA [Online] CHEMICAL ABSTRACTS SERVICE, COLUMBUS, OHIO, US; BECCALLI, EGLE M. ET AL: "A new access to diarylmaleic anhydrides" retrieved from STN Database accession no. 131:157687 XP002252140 & EUROPEAN JOURNAL OF ORGANIC CHEMISTRY (1999), (6), 1421-1426 ,
- DATABASE CA [Online] CHEMICAL ABSTRACTS SERVICE, COLUMBUS, OHIO, US; BECCALLI, EGLE M. ET AL: "A new synthesis of staurosporinone" retrieved from STN Database accession no. 129:136342 XP002252141 & TETRAHEDRON (1998), 54(24), 6909-6928 ,
- DATABASE CA [Online] CHEMICAL ABSTRACTS SERVICE, COLUMBUS, OHIO, US; BECCALLI, EGLE M. ET AL: "New indole derivatives. Synthesis of 3-acyl-1-carbethoxy-2- trifluoromethanesulfonylindoles and of substituted 2-vinylindoles" retrieved from STN Database accession no. 122:290644 XP002252142 & TETRAHEDRON (1995), 51(8), 2353-62 ,
- DATABASE CA [Online] CHEMICAL ABSTRACTS SERVICE, COLUMBUS, OHIO, US; SHI, GUO-QIANG ET AL: "Ethyl 3-Fluoro-3-(tributylstannyl)-2-methoxyacry late: Preparation and Palladium/Copper-Cocatalyzed Cross-Coupling Reactions as a Novel Route to.beta.-Fluoro-.alpha.-keto Acid Derivatives" retrieved from STN Database accession no. 124:56593 XP002252143 & JOURNAL OF ORGANIC CHEMISTRY (1995), 60(20), 6608-11 ,
- DATABASE CA [Online] CHEMICAL ABSTRACTS SERVICE, COLUMBUS, OHIO, US; KARMINSKI-ZAMOLA, GRACE ET AL: "Applicability of the Oglialoro condensation in the reaction with 3-indolecarbaldehyde" retrieved from STN Database accession no. 103:22407 XP002252144 & HETEROCYCLES (1985), 23(2), 313-16 ,
- DATABASE CA [Online] CHEMICAL ABSTRACTS SERVICE, COLUMBUS, OHIO, US; ROZHKOV, V. S. ET AL: "Indole derivatives. CII. C-Acylation of 3-indolylacetonitrile" retrieved from STN Database accession no. 82:139887 XP002252145 & KHIMIYA GETEROTSIKLICHESKIKH SOEDINENII (1974), (11), 1502-5 ,
- DATABASE CA [Online] CHEMICAL ABSTRACTS SERVICE, COLUMBUS, OHIO, US; CHATTERJEA, J. N. ET AL: "Synthesis of N-methylindolo[2,3-c]pyrans and related compounds" retrieved from STN Database accession no. 110:75350 XP002252146 & JOURNAL OF THE INDIAN CHEMICAL SOCIETY (1987), 64(11), 670-2 ,
- DATABASE CA [Online] CHEMICAL ABSTRACTS SERVICE, COLUMBUS, OHIO, US; PREOBRAZHENSKAYA, M. N. ET AL: "Effect of nucleophilic reagents on.alpha.,.beta.-disubstituted esters of 1-acetylindole-3-propionic acid" retrieved from STN Database accession no. 71:21962 XP002252147 & ZHURNAL ORGANICHESKOI KHIMII (1969), 5(4), 746-52 ,
- DATABASE CA [Online] CHEMICAL ABSTRACTS SERVICE, COLUMBUS, OHIO, US; GAIGNAULT, J. C. ET AL: "1-(Indolylmethyl)-2,3,4,9-tetrahydro-1H-. beta.-carbolines prepared from tryptamine or its derivatives" retrieved from STN Database accession no. 91:56876 XP002252148 & ANNALES PHARMACEUTIQUES FRANCAISES (1978), 36(11-12), 561-8 ,
- DATABASE CA [Online] CHEMICAL ABSTRACTS SERVICE, COLUMBUS, OHIO, US; SAKURAI, SETSUJI ET AL: "Studies on indole derivatives. A new condensed ring compound. 2-Pyrono[3,4-b]indole" retrieved from STN Database accession no. 54:7166 XP002252149 & NIPPON KAGAKU ZASSHI (1957), 78, 1665-8 ,
- DATABASE CA [Online] CHEMICAL ABSTRACTS SERVICE, COLUMBUS, OHIO, US; OKABE, NOBUO ET AL: "2-Hydroxy-3-(1H-indol-3-yl)propenoic acid" retrieved from STN Database accession no. 129:252722 XP009016130 & ACTA CRYSTALLOGRAPHICA, SECTION C: CRYSTAL STRUCTURE COMMUNICATIONS (1998), C54(9), 1330-1331 ,

## Description

### Background of the Invention

### 1. Field of the Invention

The present invention refers to the enol tautomer of 3-indolylpyruvic acid (briefly IPA) obtained in an essentially keto tautomer-free form, suitable for the employ in pharmaceutical industry treatment steps, as well as to salts thereof, particularly to alkali and of alkali-earth metal salts.

The invention further refers to a process for the production of the above cited enol tautomer and of the salts thereof in solid form, preferably of essentially crystal structure, as well as their activity as therapeutically active agents, specifically in the treatment of central nervous system disturbances.

### Description of the prior art

Keto-enol tautomerism is a well-known chemical phenomenon, definable as the entirely reversible migration of a Hydrogen atom from a Carbon in alpha, adjacent to a carbonyl group, with a subsequent double bond shift. The forms thus generated have distinguishable chemico-physical features, and are respectively defined as keto and enol 'tautomers'. In a solution, all keto species, alpha-ketoacids included, are subjected to dynamic transformation of a tautomer into the other until attaining an equilibrium situation, whose extent depends on factors such as the chemical species considered, the solvents, the pH, the temperature, etc. However, tautomers, being intrinsically transformable into each other with the utmost ease, are not usually treated as chemical species *per se,* i.e. capable of giving rise to clearly distinct biochemical reactions, being instead generally assessed for their end effects in solutions, as if being a single compound.

In the case of stereoisomers, a spontaneous conversion of the isomeric forms into each other is normally impossible to occur. In particular, if a first of the forms is the therapeutically active one, the other enantiomer is considered as an impurity, which can be eliminated by conventional purification methods.

On the contrary, in the case of the tautomers, these convert into each other spontaneously even under mild conditions, until they arrive at a balance situation. In fact, the tautomers are a dynamic system in a continuous and macroscopic modification depending on the experimental and preservation conditions. Consequently, while it is still possible to state by conjecture that only one of the ' tautomeric forms is that therapeutically active, the other tautomer can not simply be considered as an impurity to be eliminated, but as a species spontaneously taking part to the structural balance. By this spontaneous mechanism of action, the "concentration" of the therapeutically active species will always be made uncertain.

3-indolylpyruvic acid (3-indolylpropionic-α-oxo acid) is an alpha-ketoacid widespread in the vegetable and animal world. It being the transamination product of tryptophan, an amino acid of the natural series, essential in the diet of several animals and found in most proteins, the former is certainly present in all living cells, though its quantitative determination has always entailed several technical problems.

For instance, for several decades now it has been known that 3-indoleacetic acid, acting as growth factor in plants, directly derives from the decarboxylation of 3-indolylpyruvic acid, which in turn is obtained by transamination from tryptophan. However, the studies conducted to quantify the extent of these transformations had serious difficulties: firstly, the various methods used to synthesize 3-indolylpyruvic acid led to multicolored compounds, whose chemico-physical behaviour proved erratic and depending on the specific synthesis method used; secondly, the presence of the keto-enol tautomerism hindered the clear identification of the chemical species originated by tryptophan transamination. Several decades ago, in an attempt to understand the features of the keto-enol tautomerism of 3-indolylpyruvic acid many basic works were conducted, which yielded highly interesting results, above all considering the limitations in the instrumentation available at that time.

E.g., J.A.Bentley et al. (Biochemical J. 64, 44-49, 1956) demonstrated that freshly synthesized 3-indolylpyruvic acid behaves in a wholly different manner in paper chromatography, depending on the systems used as eluent: a solution consisting of isopropanol, ammonia and water completely destroys the alpha-ketoacid, whereas the acetic acid-water mixture allows to obtain two distinct blots. Always using paper chromatography, Schwarz and Bitancourt (Biochemical J. 75, 182-7, 1960) demonstrated 3-indolylpyruvic acid to be an extremely unstable substance that cannot be adequately singled out in chromatograms, either because it is completely destroyed, or because the solvents used do not separate it from its decomposition products: adopting proper care and a bidimensional chromatogram, two distinct products could be obtained, deemed to be the keto and the enol tautomers, plus several other unidentified decomposition products. In the same work, it is reported that probably the ketoacid precipitates in a mainly enol form, yet in a solution at pH 8.0 it was largely converted to the keto form in 20 minutes. Schwarz (Archives Biochem.Biophys. 92, 168-75, 1961) described the separation by paper chromatography of the two tautomers of 3-indolylpyruvic acid, followed by UV spectrum identification: the typical spectrum of the enol tautomer gradually converted, in over 2 hours, to that of the keto tautomer, whereas the spectrum of the keto form rapidly converted to that of the enol form, in the presence of boric acid and of tautomerase (an enzyme facilitating interconversion between the two forms). In two accurate works, J.M.Kaper et al. described studies on 3-indolylpyruvic acid conducted by paper chromatography (Archives Biochem.Biophys. 103, 469-74, 1963) and on their UV spectra (Archives Biochem. Biophys. 103, 475-87, 1963). They reported that in ammonia environment the paper chromatography of 3-indolylpyruvic acid rapidly leads to a decomposition toward at least 7 products of indolic nature, of which only a few were identified. The work concludes stating that, under the conditions used, a rapid conversion from the enol to the keto form was apparent. However, the reaction subsequently progressed to degradation products, stimulated by the presence of oxygen, light, ammonia, or even of solvent contaminants. Concerning the spectrophotometric studies, they confirmed that, while 3-indolylpyruvic acid in solid form is mostly found as enol, when dissolved it assumes an equilibrium situation that always favours the keto form. Moreover, pH seems to markedly influence the keto-enol equilibrium, since in the acid region (pH 2.9-6.0) the enol form is reasonably stable, whereas in the basic region (pH 7.7-9.5) the spectrum of the keto form rapidly predominates. Lastly, Nazario and Schwarz (Archives Biochem.Biophys. 123, 457-61, 1968) conducted studies on the keto-enol equilibrium of 3-indolylpyruvic acid, using IR spectrometry. They reported that, while the enol form of the ketoacid predominates in the solid form, in acid environments, in aprotic solvents or in methanol, the keto form predominates in non-acid aqueous solvents and in the presence of protophilic solvents.

Therefore, overall, the studies published in the '50s and in the '60s lead to conclude that 3-indolylpyruvic acid is a compound characterized by great instability, precipitating mainly in the enol form. However when the solid is dissolved in an aqueous solution, the equilibrium is swiftly shifted to the keto tautomer and in physiologic solution "in vitro" the specific pattern of the enol tautomer disappears after a few minutes. In fact, in aqueous solution at neutral or basic pH IPA rapidly reaches an equilibrium in which the keto form markedly predominates. Accordingly, the ketoacid tautomers should be considered indistinguishable in their physiopathological effects on plants and animals, since, due to the the fixed temperature and pH conditions in the cell environment, the equilibrium reached in solution between the tautomers of 3-indolylpyruvic acid is always characterized by the marked predominance of its keto form, and this will determine the metabolic destiny of the ketoacid.

Therefore, according to the prior art teaching, it was irrelevant to have a lower or higher proportion of enol tautomer, in that it was expected that "in vivo" only the keto tautomer would have been present.

### Technical problem of the invention

In the Technical Abstract Database with accession number 131:157687, an article by Becalli, Egle M. et al. XP002252140, headed

"A new access to diarylmaleic anhydride" describes a derivative of 3-indolyl pyruvic acid whch is constituted of the enol and keto tautomer forms of the 3-indolyl pyruvic acid.

EP-A-0 421 946 discloses derivatives of 3-indolylpyruvic acid and their use in the treatment of e.g. ischemia, epilepsy and Alzheimer's. OKABE, NOBUO ET AL (Acta Crystallographica Section C, 54, 1330-1331, 1998) discloses the crystal structure of the enol tautomer of 3-indolylpyruvic acid and suggests that the planar conformation of the enolic form of indole-pyruvic acid may be considered as an enzymatically recognizable form.OKABE ET AL and the prior art cited therein disclose 1) that 3-indolylpyruvic acid has an anxiolytic activity in mice, 2) that it is known to regulate the modulation of brain kynurenic acid content and 3) that it plays a role in the metabolism of patients with phenylketonuria.

The literature on 3-indolylpyruvic acid reports, already from the '50s and '60s, that the ketoacid, obtained via different synthesis methods in organic solvents, precipitates mainly in the enol tautomeric form. However, it has been observed that the presence of impurities (consisting of both the keto tautomer and the other compounds obtained as secondary products in synthesis processes) represents a critical factor in determining the chemico-physical features of the powders obtained, as well as the metabolic destiny of the molecular species in the solution, hence outmost care should be exerted in obtaining enol form at the maximum purity grade theoretically attainable.

Moreover, as a result of the present invention and the pharmacological tests carried out with the products thereof, it has surprisingly been found that the sole enol form of 3-indolylpyruvic acid, thus excluding the keto form, is accountable for the favourable pharmacological results linked to the therapeutic use of 3-indolylpyruvic acid. Hence, the stability of the enol form becomes a critical factor in the pharmaceutical utilization of 3-indolylpyruvic acid.

Furthermore, based on the present invention, it has been found that:
a) contrary to what occurs "in vitro", human and animal biologic fluids treated by the enol tautomeric form show that the enol tautomer is stable for many hours (likely by action of tautomerases);
b) the keto tautomer form is practically devoid of any pharmacologic activity, whereas the the enol form shows a wide pattern of activity useful in therapy,
c) the salification of the enol form allows the use of the enol compound also through an injection route of administration, when it can not be administered orally.
d) further to the practically pure enol form of IPA ( enol purity 99,9%), it has been found that the enol form itself as obtained with the process of the present invention, surprisingly consists exclusively of the (Z) geometric isomeric form of the enol tautomer, instead of being a mixture of the two possible geometric isomer forms (E) and (Z).

Consequently, obtaining the enol tautomer of IPA in the state of highest purity both of the enolic and the (Z) form of isomers, makes possible to attain foreseeable and reliable therapeutic effects, contrary to the case in which also the keto tautomer was present from the beginning.

Hence, an object of the present invention is to obtain the enol form of 3-indolylpyruvic acid in a physical state allowing a reliable pharmaceutical use on the drug manufacturing stage and a reliable therapeutic utilization of said drugs. Therefore, the object is that of obtaining the enol tautomer form in a semicrystalline ` solid state and in a virtually pure state, essentially free from the keto form of 3-indolylpyruvic acid. This means that such virtually pure state is equivalent to a theoretical resolution of the enol form with respect to the keto-enol tautomerism of 3-indolylpyruvic acid.

The prior art reports several methods for obtaining 3-indolylpyruvic acid: apparently, some methods (e.g those described in U.S. Pat. 4,551,471 and in U.S. Pat. 5,002,963) are not useful to obtain solely the enol tautomer of 3-indolylpyruvic acid, as there are also obtained large amounts of the keto form of 3-indolylpyruvic acid, from which it is not possible to obtain the corresponding high-purity enol form anymore.

Other methods described (es. Hoppe-Seylers z. 109, 259, 1920; Biochem J. 64, 44, 1956; Arch.Bioch.Biophys. 103; 469, 1963; J.Biochem. 44, 47, 1957; U.S. Pat. 5,210,215) allow to obtain more than the 90% of enol form, yet the compounds are often polluted by residues of other substances of indolic nature and, above all, by colored substances whose chemical characterization is virtually impossible, and deriving from free radical capture phenomena and subsequent polymerization phenomena.

U.S. Pat. 4,808,728 to the same Applicant also teaches a process for the production of 3-indolylpyruvic acid. The product is obtained with a high purity grade, yet such process fell short of providing the results advanced by the present invention. Moreover, the product obtained has a structure of flocculent nature, instead of a semicrystalline structure, being therefore quite unsuitable for pharmaceutical processing and of uncertain stability.

US patent 4 808 728 describes a synthesis method of preparation of indolyl pyruvic acid (briefly IPA). The tautomeric features thereof are not specified in that patent disclosure. The recovered acid has a good purity level (absence of polymerization products, other substances of indole structure and color), and an acceptable chemical recovery yield of about 50%, with an amorphous structure of the solid precipitate product.

The above mentioned method was a useful starting point on the way to the present invention. However in the present invention, a plurality of modifications to the synthesis procedure of US patent 4 808 728 have been made. A remarkable yield increase in the recovery (higher than 80%) of IPA in its (Z)-enol tautomeric form has been obtained with an enol purity higher than 99,9% and a recovered solid precipitate of semicrystalline structure having an industrially useful shelf life.

Furthermore, it has been surprisingly found that while can exist two possible geometric isomers of the enol tautomeric form of IPA, which are the geometric E and Z isomers, exclusively the geometric isomer Z is formed in the synthesis of the present invention.

This result will be evidenced by the data of 1-H and 13-C NMR analysis of the product, which will be shown later in this description.

The results obtained with the present invention can be summarized as follows:
a)only one of the two tautomeric structures is formed (the enolic form) with high yields in chemical conversion,
b)the enolic form obtained consists exclusively of the geometric isomer Z of IPA,
c)this Z form is a precipitate having the highest level of chemical purity,
d)the physic state of the precipitate is a semicrystalline structure, highly homogeneous in structure and color and of easy workability and recovery.
e)the solid precipitate has a high shelf life structural stability (absence of both a spontaneous conversion of one tautomer into the other with no formation of degradation products) when maintained in the normal preservation conditions of a pharmaceutical raw material.

In connection to the above, it is to point out that geometric isomers (E) and (Z) generally due to the presence of a double bond, as occurring in the enolic IPA structure, can usually have even very different chemico-physical properties. As an example, one can consider the extreme case of difference between fumaric acid (geometric (E) isomer) and maleic acid (geometric (Z) isomer).

Therefore the teaching deriving from prior art US patent 4 408 728 was that in the method of production of IPA a spontaneous tautomeric balance would occur between the keto and enol tautomeric forms. The enol form, in turn, would find an a priori unforeseeable balance between the percentages of the two geometric isomers (E) and (Z) respectively.

As a consequence of the possible differences in the chemico-physical properties of the geometric isomers, also the pharmacological properties thereof should be expected to be different.

In view of the above, the IPA produced according to the prior art was comprising a mixture in variable proportions of the two tautomeric forms and of the two geometric isomers. This resulted in a not clear pharmacological profile of IPA as well as in unreliable results of the pharmacologic tests.

In the present invention a IPA is provided consisting of only one tautomeric form never individually insulated in the prior art in a stable and pure state. In addition the enolic form as obtained consists of only one precisely identified geometric isomer form, not known in the prior art as a pure and stable individually resolved enantiomer. This product is obtained at the highest chemical purity level, and moreover it has the unexpected property of a shelf life comparable to the usual pharmaceutical raw materials. This now allows for the first time in the pharmacological art to carry out tests on IPA with expectance of unambiguous and reliable results.

According to the present invention, 3-indolylpyruvic acid in a virtually pure enol form (≥99% purity, absence of keto form and of polymerization products) is obtained for carrying out the subsequent biochemical and pharmacological tests with certainty of product stability.

A further object of the present invention is a process providing the product in a nearly entirely needle-shaped crystal form, anyhow wholly suitable to be employed by the pharmaceutical industry and for the preparation of capsules, tablets, injection ampoules.

In above mentioned US patent 4 808 728 (Example 4) high care was taken to maintain a reaction environment anhydrous as much as possible. In fact the focal point in the transformation of tryptophan into IPA was the condensation reaction of the aromatic aldehyde with the amine function, the fundamental water removal of water from the adduct and the rearrangement thereof from aldo-amine to keto-imine.

In view of the above preliminary assumptions the use clear of "powerful" reactants, such as 1,8-diazabicyclo(5.4.0)-undec-7-ene (briefly DBU) as a dehydrating non-nucleophilic base and anhydrous ZnCl₂ as a complexant agent as well as the operation under controlled temperature and "stiff' reaction times.

Thus, the procedure of the cited prior art is carried out under "forced" conditions and reactants. This brings the consequence of a relatively narrow window of operating conditions.

On the contrary, the method of the present invention starts from a different philosophy based on a better manageable "soft" approach, which will be hereinafter disclosed in comparison to the cited US patent 4 808 728. In this discussion, the particular distinguishing features are pointed out which have brought to the far superior results obtained with the present invention.

### A) Use of triethylamine (TEA) in lieu of DBU.

Advantages: reduced heat, better temperature and reaction time control, no need of anhydrous solvents, a weaker base and so reduced side reactions, lower cost, lower toxicity, more soluble intermediate salts.

Both DBU and TEA are tertiary aliphatic non-nucleophilic bases. However they have remarkably different basic characteristics (higher for DBU), while they ' show also a remarkably different volatility (higher for TEA). In view of this in a synthetic procedure development usually DBU is preferred to TEA, rather than the contrary. With the present invention it was found that TEA could perform the role of DBU with the advantage of a minor chance of side reactions.

In addition TEA allows an easier and precise temperature control. It was in fact discovered that an excessive and uncontrolled temperature of the reaction mixture at this procedure step reduced the IPA yield and quality.

### B) Use of Zn acetate bihydrate in lieu of anhydrous ZnCl₂.

In the prior art procedure the use of anhydrous ZnCl₂ as a complexant and in part dehydrating agent is consistent with the philosophy of "forcing" the reaction mechanism and this necessarily implies the use of an anhydrous and controlled reaction environment.

In view of its properties, anhydrous ZnCl₂ was considered particularly suitable to the desired development of the reaction mechanism. It showed however also manageability and treatment difficulties in operation not compatible with the scale-up of IPA synthesis according to the "soft" philosophy of the present invention.

It was found that using of Zn acetate bihydrate results in a substantial improvement of operation, yield, and quality of the IPA precipitate. This improvement is shown both by the bihydrate and the anhydrous form of the acetate salt. This permits the use of solvents not necessarily anhydrous, provided that they have the minimum water content.

In addition, zinc acetate bihydrate can be found on the market under a dosable non-deliquescent crystalline form which makes a stechiometric control of the reaction easier. It shows practically no solubilization heat in methanol, this also being in favour of the reaction control.

It is to emphasize that it is a surprising and unobvious issue of the present invention the rendering possible the use of a hydrate salt to make an intermediate product soluble, while the prior art teaching supposed that the salt had to be anhydrous for a successful result of the process. In addition it is to note that this is obtained without changing the stechiometry of the dehydrating base and even substituting it with a "weaker" one (TEA).

### C) Use of picolyl aldehyde (PCA) in lieu of isonicotin aldehyde (ISNA).

Advantages: improved stability on time; improved purity (PCA>99%, ISNA max 97%); improved reaction and timing control; much higher IPA recovery; much improved IPA quality.

The above cited US patent 4 808 728 uses ISNA for the reaction of the amine function of tryptophan, the Shiffs base formation, the rearrangement thereof from aldoimine to ketoimine. This was made possible by ISNA having the carbonyl-pyridine nitrogen substituents in a 1,4- (or para) position.

PCA is a pyridine substituted in orto position (or 2-position) with a carbonyl group of aldehyde type.

By using PCA in lieu of ISNA, on a theoretical basis, it should be expected an even more "forced" reaction condition and more unstable intermediate products of the reaction. In fact it is neither spontaneous nor easy to attain a rearrangement process of the aromatic structure which would result in the formation of a structure of quinoid-1,2 type. At a first sight PCA should not appear a good substitute for ISNA. This opinion would be also confirmed by the higher instability of ISNA in comparison to PCA, so that ISNA should appear more reactive than PCA.

Contrary to the above, according to the present invention, PCA results superior to ISNA in view of the stability of the various reaction intermediates and the relative stability thereof. In fact, the stabilization by formation of a Zn complex (in particular with Zn acetate which turns out to be more effective than chloride) resulted in a more efficient and organized process intermediate (in favor of energy) with a mechanism of formation completely different from ISNA.

Briefly, this aspect of the present invention makes possible to control the course of the reaction by minimizing any interfering products and thus obtaining the highest of yields and definitely improving the quality of IPA.

### D) Use of non-anhydrous solvents.

Advantages: Cost abatement; improved control of reaction (no need of maintaining anhydrous conditions); simplified procedures.

While the use of anhydrous solvents is compulsory in the method disclosed in US patent 4 808 728, the use of non-anhydrous solvents in the present invention is a logical consequence of the discussion above. This a further confirmation of the "soft" philosophy at the basis of the present invention, in contrast to the method described in the cited prior art document.

In more recent years, 3-indolylpyruvic acid was the subject of several pharmacological and clinical studies, as well as of some invention patents, in order to clarify its potential uses in experimental studies on animals and in human therapy. E.g., U.S. Pat. No. 4,551,471 describes a process for the biosynthesis of 3-indolylpyruvic acid via tryptophan transamination reaction, and an increase in serotonin levels in the brain, along with evident pharmacological effects, when the ketoacid was administered to rats. U.S. Pat. No. 5,002,963 describes a new method of biosynthesis of 3-indolylpyruvic acid, based on the use of L-aminoacid oxydase on tryptophan, as well as the remarkable capability exhibited by the ketoacid to directly transform in kynurenic acid, and therefore to antagonize the toxic and the degenerative effects due to excitatory amino acids. U.S. Pat. No. 5,075,329 illustrates the powerful antiradical properties of 3-indolylpyruvic acid, as well as its potential pharmacologic applications in cardiopathies, inflammations, arteriosclerosis, etc. U.S. Pat. No. 5,091,172 describes the antioxidizing and antimutagenic properties of 3-indolylpyruvic acid, and its potential use in cosmetics, for protecting skin from oxygen, sun and aging. U.S. Pat. No. 5,210,215 illustrates a new method of chemical synthesis of 3-indolylpyruvic acid and of its derivatives, useful in degenerative diseases, based on the use, as starting compounds, of benzenes or indoles substituted in the benzene ring. U.S. Pat. No. 5,447,951 describes the 3-indolylpyruvic acid capabilities of acting as antistress agent, reducing corticosterone levels in rat.

Concerning pharmacology studies, Bacciottini et al. (Pharmacol.Res.Commun. 19, 803-17, 1987) described 3-indolylpyruvic acid to increase cerebral serotonin turnover in rat, and to possess sedative and analgesic activities. Russi et al. (Biochem.Pharmacol. 38, 2405-9, 1989) demonstrated a marked increment of kynurenic acid in all tissues of rats, following administration of 3-indolylpyruvic acid, suggesting a new metabolic pathway for the formation of this important inhibitor of the excitatory amino acids. Squadrito et al. (J.Cardiovasc.Pharmacol. 15, 102-8, 1990) described the anti-hypertension effects of 3-indolylpyruvic acid in different rat hypertension models, relating them to the increase in the brain concentrations of serotonin. Merlo Pich et al. (Acta Physiol.Scand. 139, 583-9, 1990) described the effects of the ketoacid on sleep and on food ingestion in rat, demonstrating that while the anorexigenic effects disappear after a few days of treatment, the sleep enhancing effects are maintained even for chronic treatments. Ferretti et al. (Eur.J.Phannacol. 187, 345-56, 1990) described the effects of 3-indolylpyruvic acid as stimulator of melatonin production in rat pineal gland. Bruno et al. (Neurosci.Res.Commun. 8, 137-46, 1991) demonstrated that 3-indolylpyruvic acid and its derivatives can act as antagonists both on the receptor of the kainic acid and on the NMDA ones, attenuating stimulation of calcium uptake in cerebellar neuron cultures. Squadrito et al. (Neurosci.Res.Commun. 9, 27-36, 1991) described the reduction effects of 3-indolylpyruvic acid on food assumption by genetically obese rats (Zucker). Biagini et al. (Biol.Psychiatry 33, 712-9, 1993) analyzed the effects of 3-indolylpyruvic acid on corticosterone plasma levels and on the glucocorticoid hippocampal receptors, when the rats were subjected to a set of stressor events. Zoli et al. (Neurochem.Int. 23, 139-48, 1993) reported the effects of 3-indolylpyruvic acid in an experimental ischemia model in rat. Lapin and Politi reported the activities of 3-indolylpyruvic acid in mouse, both as anxiolytic agent (Pharmacol.Res. 28, 129-34, 1993) and as inhibitor of the toxic effect of ethanol (Alcohol & alcoholism 29, 265-8, 1994).

Concerning clinical studies, Silvestri et al. (J.Intl.Med.Res.19, 403-9, 1991) reported that six healthy volunteers, treated for some nights with doses of 3-indolylpyruvic acid ranging from 100 to 200 mg, exhibited improvements in sleep similar to those observed with tryptophan or with melatonin. Shamsi et al. (Human Psychopharmacol. 11, 235-9, 1996) investigated the effects of 3 doses of 3-indolylpyruvic acid (100, 200 and 300 mg) in 10 young volunteers having sleep problems, reporting that ketoacid has a bland action on sleep, probably mirroring a more marked intervention on stress attenuation.

It is important to underline that, in the works published in the '50s and '60s, the researchers interested in 3-indolylpyruvic acid appeared divided by several contrasts and controversies, since results obtained by one team were seldom confirmed by others: this was due above all to the different methods used to synthesize the ketoacid, as even small amounts of impurities could lead to rapid deterioration of the substance, and to fundamental differences in its behavior in a solution. It is also important to underline that, whilst all involved researchers were aware of the presence of the tautomers when 3-indolylpyruvic acid was solubilized in aqueous solutions, in all the works reported in literature no reference is ever made to specific precautions to be adopted in the ketoacid solubilization. This is due to the fact that, owing to the supposedly easy interconversion, an equilibrium between the two tautomers is soon reached, at last determining the physiological and pharmacological end effects.

Object of the present invention is the enol tautomeric form of 3- indolylpyruvic acid in a chemically stable, semicrystalline, solid state, having an enol purity not lower than 99% referred to the total acid.

### Summary of the invention

A further object of the invention is the enol form of IPA which further consists exclusively of the (Z) form of its geometric isomers formed around the double bond.

An additional object of the present invention is the above recited form of IPA for therapeutic use at the level of central nervous system, cardiovascular pathologies, and treatment of neuron cells and tumors.

Still an object of the invention is a process for the production of the enol tautomeric form of 3-indolyl-pyruvic acid, comprising reacting the amino function of tryptophan with an aromatic aldehyde, in the presence of a complexant agent and a proton acceptor amine base, in a reaction solvent and hydrolyzing the product of said reaction in a hot acidic hydrolysis step at a temperature of 45 to 65 wherein: the aromatic aldehyde is 2- pyridine-carboxyaldehyde alone or in a mixture with triethylamine, the proton acceptor non-nucleophilic amine base is triethylamine , the complexant agent is selected from the group consisting of zinc acetate or zinc sulfate, and the organic polar solvent as a reaction solvent is selected in the group consisting of tetrahydrofuran, dimethyl-formamide, acetonitrile, and methanol.

Object of the invention are also alkaline or earth-alkaline salts of the enolic tautomer of IPA, alkaline or earth-alkaline salts of the enolic tautomer of IPA for the same therapeutical uses as that of enol IPA and a process for the production thereof comprising the step of neutralizing said enol tautomer of 3-indoly-pyruvic acid with a neutral one of said alkaline or earth-alkaline metals, in a first aprotic polar solvent, together with a second protic polar solvent as a metal activator.

An additional object of the invention is the use of the above mentioned compounds for the pharmaceutical industry, in that for the first time those compounds are in a form which can be employed in the industry in view of their reliable composition, manageable semicristalline structure and shelf life stability.

As an enol IPA derivative is also an object of the invention the indolyl lactic acid which is in an interconversion equilibrium with the claimed enol IPA componds, so that IPA can behave as a precursor of lactic acid or viceversa.

Pharmaceutical compositions comprising the above mentioned compounds are also an object of the invention.

In the light of the pharmacological tests carried out with the enol tautomer of the present invention, it has now been found, according to another aspect of the present invention, that:
1) The conversion between the two tautomers of 3-indolylpyruvic acid is only partially reversible, and the keto form, originating in aqueous solutions from the enol, mainly follows metabolic pathways autonomous and independent from the latter;
2) the enol tautomer form is the form mainly accountable for the relevant biochemical and pharmacological properties, described in literature when 3-indolylpyruvic acid was used;
3) the enol tautomer form appears particularly stable in biological tissues, owing to the presence of specific enzymes (tautomerase), which hinder conversion to the keto form.

Disclosed are also the applications for therapeutic use of the enol in form essentially free from keto tautomer, previously ascribed to the tautomer mixture, exploiting the easy dissolving in physiological environments of enol and of its salts, the determination of the enol tautomer concentrations in animal tissues and plasma, and lastly the biochemical and pharmacological effects attained when the tautomer, or its salts, are administered to experimental animals or to human cell cultures.

### EXPERIMENTAL PART

### 1. Synthesis of the enol tautomer of 3-indolylpyruvic acid and of its salts

**A**) process for the production of the enol of 3-indolylpyruvic acid through a reaction of tryptophan and 2-pyridine carboxy aldehyde in the presence of a complexing agent and of triethylamine as amine base in a polar organic solvent, and hot hydrolysis of the reaction product.
**B**) preparation of alkali and alkali-earth metal salts, prepared by direct or indirect neutralization of the enol of 3-indolylpyruvic acid, yielded by the synthesis described in A).

In accordance with the invention, hereinafter the qualifying data of the syntheses identified in A) are described.

As aromatic aldehyde, preferably 2-pyridinecarboxaldehyde, also called picolinaldehyde or PCA, is used, under conditions of excess with respect to Tryptophan, or of slight defect, compensating the lacking amount with a tertiary amine base. Above all the second combination leads to a substantial increase in the recovery yield of the enol of 3-indolylpyruvic acid, also by virtue of an improved control of the reaction temperatures, due to the fact that PCA develops less solubilization heat.

As complexing agent various organic and inorganic zinc salts have optionally been used, hydrate as well as anhydrous, also dissolved in an organic solvent, not necessarily the same one of the coupling reaction. An exemplary selection is: zinc sulfate, zinc acetate (anhydrous as well as hydrate) and zinc chloride dissolved in anhydrous tetrahydrofuran. In particular, the best recovery yields are attained using hydrate zinc acetate, previously vacuum stored on a drying agent such as, e.g., potassium hydroxide (KOH) pellets. Moreover, the hydrate zinc acetate, thus stored, forms, with the intermediates of the coupling reaction, complexes that are more soluble than those of the other salts.

As proton acceptor and dehydrating tertiary amine base triethylamine was preferentially used, forming intermediate salts more soluble with respect to the corresponding ones of other known bases having similar features, like DBU or other trialkyl alicyclic bases (DIEA and the like). Moreover, with respect to the indicated bases, triethylamine develops less solubilization heat, allowing an improved control of the run of the reaction temperature, above all at the critical step of dehydrating, and the entailed structural rearrangement of the coupling intermediate.

As reaction solvent polar solvents are used, not necessarily anhydrous, like DMF, acetonitrile, THF or alcoholic solvents having a short aliphatic chain, because of the improved solubilization and solvation of the reaction intermediates and the maximum solubility at the end stage of high-temperature acid hydrolisis. In particular, the best performances are attained using methanol.

The reaction may be conducted at room temperature or, preferably, at low temperature, at which the best results are obtained, according to the specific combination of reactants used. To improve the quality of the enol precipitate of 3-indolylpyruvic acid, the acid hydrolisis process should be conducted under hot conditions at 45 to 65°C, preferably at 55°C. The acid environment should be for a mineral acid, preferably 1N hydrochloric acid, diluted with a suitable amount of distilled water.

Overall, with respect to the preparations of 3-indolylpyruvic acid described in the above cited patent, the process for the preparation of the enol of 3-indolylpyruvic acid object of the invention entails a substantial improvement of the end recovery yields (>80%), a higher purity in enol form (>99.9%), an improved quality of the features of the precipitate (pale yellow color, near-crystal consistence) and the consequent improvement in the pharmaceutical processability of the solid. Moreover, these features make this precipitate particularly suitable for the subsequent preparations, described in the invention, of alkali and alkali-earth metal salts. The latter were prepared with the dual purpose of making the enol form of 3-indolylpyruvic acid immediately soluble in saline and of detecting potential salt-specific pharmacological activities.

In accordance with the invention, hereinafter the qualifying data of the syntheses, identified at B), are described.

The preparation of the salts of the enol of 3-indolylpyruvic acid involved alkali and alkali-earth metal salts, and in particular those of Sodium, Lithium, Potassium, Magnesium and Calcium.

The salts were prepared by neutralization of the enol of 3-indolylpyruvic acid and of a suitable chemical form of the alkali and alkali-earth metals described in the invention, in a suitable anhydrous non-protic polar solvent, in particular diethylic ether or tetrahydrofuran, in an amount such as to ensure both the complete solubilization of the indole alpha-ketoacid and the complete precipitation of the generated salt.

In particular, the alkali and alkali-earth metals were used as neutral metal (when possible) together with a set amount of an anhydrous protic polar solvent, specifically methyl alcohol or ethyl alcohol, as metal activator.

In particular, the alkali and alkali-earth metals were used in the chemical form of the corresponding methoxides or ethoxides, when possible, using a known-titre alcoholic solution in an alcoxide and calculating the adequate amount of alcoholic solution required for the exact neutralization of the enol of 3-indolylpyruvic acid, in an amount by volume of non-protic solvent such as to ensure the quantitative precipitation of the generated salt.

### Report on structural analysis of indolyl 3-pyruvic acid (IPA) carried out through NMR spectroscopy.

The IPA structure has been determined by means of NMR spectroscopy, by performing proton (1-H: 200 MHz) and Carbon 13 (13-C: 50,3 MHz) spectra in deuterated tetrahydrofuran (THF - d8).

All NMR signals related to the nine hydrogen and eleven carbon atoms of the molecule have been detected and allotted to the specific positions of the IPA molecule, which is present in THF-d8 (as well as in deuterated methanol) as 100% enolic tautomer, in the unique (Z) type configuration.

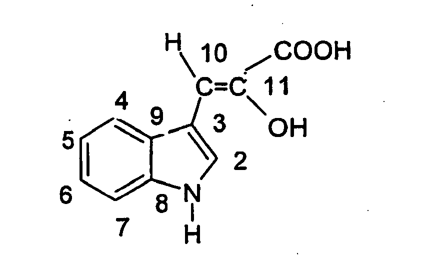

### 1) - First set of spectra.

A set of proton spectra have been performed at a 0,5 mg/ml concentration in THF-d8. In these conditions all the signals relative to the nine hydrogens (protons) of the molecule can be clearly observed.

In particular the following allotments can be assigned:

| | |
|---|---|
| COOH | 11,25 ppm |
| NH | 10,45 ppm |
| OH | 7,75 ppm (doublet by H₁₀,OH - J=1,8 Hz coupling |
| H₁₀ | 6,85 ppm (doublet by H₁₀,OH - J=1,8 Hz coupling) |
| H₂ | 7,93 ppm (doublet by H₂,NH - J=2,7 Hz coupling) |
| H₄ | 7,64 ppm (with only one orto coupling) |
| H₇ | 7,32 ppm (with only one orto coupling) |
| H₆ | 7,12 (with two orto couplings) |
| H₅ | 7,02 (with two orto couplings) |

The allotment of the positions relative to the pair of signals H₄ and H₇ and to the pair of signals H₅ and H₆ is based on the analogy with the spectrum of indole itself (P.J. Block, M.L. Heffernan, Austr.J.Chem. (1965), *18*, 353). The high coupling constant H₁₀,OH suggests that H₁₀ is in trans to OH (Z configuration). Additional spectra evidences are shown in paragraph 7 of this report. On the whole the spectra are consistent only with the enolic tautomer with 100% purity.

### 2) Second set of spectra

To the solution of the first set above DO₂ (deuterated water) was added. A disappearance of the COOH and OH proton signals due to the isotopic substitution and at a same time the appearance of the H₂O signal is observed. Moreover, the disappearance of the H₁₀,OH constant confirms the previous allotment. As the NH proton signal is less mobile, it does not disappear by deuterium exchange quickly.

### 3) Third spectra set.

The same sample as the second set, but recorded some days later, is used. The disappearance also of the NH proton signal is observed, as well as the resulting disappearance of the H₂,NH coupling. Furthermore while the signals allotted to protons H₅, H₆, H₇ do not change with respect to the second set, the ones allotted to H₄ become narrower, thus showing the disappearance of a small coupling constant (about 0,8 Hz) which is known to be present in indoles.

### 4) Fourth spectra set

### Proton spectra in THF-d8 with concentrated IPA solution.

These are substantially similar to the first set with some shift displacement (particularly the COOH group) due to the different concentration. Right in view of this the OH mobile protons exchange more quickly with each other and with the mobile protons of COOH causing a widening of the corresponding signals and again the disappearance of the coupling constant H₁₀,OH.

### 5) Fifth spectra set

### 13-C spectra of the solution prepared for the fourth set.

The eleven signals corresponding to the eleven carbon atoms are observed. The signals of lower intensity (five signals) are associated to the quaternary carbons and can be so allotted:

| | |
|---|---|
| COOH: | 169,0 ppm |
| C₈ | 138,5 ppm |
| C₉ | 129,5 ppm |
| C₃ | 112,9 ppm |

The signals allotted to C₃, C₈ and C₉ have been identified by analogy with the indole spectrum (C. Levy, R. Lichter, G.L. Nelson "NMR Spectroscopy" 2nd Edition, Academic Press (1980), page 1213). The C₁₁ allotment to 140 ppm is attributed by exclusion and fulfills the allotment of all five quaternary carbon atoms.

Again by analogy to the indole spectrum the signals of the following CH can be allotted:
C₄, C₅ and C₆: range 123,3 -121,6 -120,2 ppm,
C₇: 113,4 ppm.

The signals of the four carbon atoms C₄, C₅ and C₆ are too near to each other, both in indole and the IPA itself, to allow an individual allotment. Moreover the presence of the substituent in 3-position of IPA can modify the trend and invert some allotments in comparison to indole. For a reliable allotment the spectra described in the following set have been performed.

### 6) Sixth spectra set

The same solution as employed in the fifth set was used to record a bidimensional spectrum which correlates the proton spectrum, through the Carbon-Hydrogen direct coupling factor, to the 13-C spectra (HETCOR spectra).

Hence, by knowing the proton spectrum, it has been possible to fulfil the CH allotment as follows:

| | | |
|---|---|---|
| 105,7 ppm | C₁₀ | (it correlates with H₁₀ at 6,9 ppm) |
| 129,8 ppm | C₂ | (it correlates with H₂ at 7,9 ppm) |
| 120,25 ppm | C₄ | (it correlates with H₄ at 7,8 ppm) |
| 121,6 ppm | C₅ | (it correlates with H₅ at 7,02 ppm) |
| 123,9 ppm | C₆ | (it correlates with H₆ at 7,12 ppm) |

In addition it is confirmed the correlation of C₇(113,4 ppm) to H₇ (7,35 ppm).

Thus the sequence of all the carbon atoms is not ambiguously allotted.

### 7) Seventh spectra set

This set shows the carbon spectra fully coupled with the various hydrogen atoms, in comparison to the normal uncoupled spectra (see the fifth set).
a) Six carbons become doublets (with a further fine structure) with coupling constants C-H>= 150 Hz, confirming that they are sp²-hybridated CH. The other five carbon atoms, which are quaternary carbons, have only very small couplings.
b) The COOH signal at 169 ppm is a doublet (J=3,7 Hz) due to the coupling with H₁₀. It is known that in substitued ethylenes the carbon-hydrogen couplings separated by three bonds (type J_{CCCH} factors) are in the order of 3 - 8 Hz if they are in a *cis* configuration and 8 - 14 if in a *trans* configuration. When electronegative groups are present, as in the case of IPA, the values of the coupling factor correspond to the lower limits of the indicated ranges (A.W.Douglas, Org. Magn. Reson.;(1977)9,69; H. Vogali, W. V. Philipsborn, Org. Magn. Resn.;(1975) 7, 617).As an example in compound CH₃(AcO)C=CH₂, which has an oxygen linked to the double bond as in the case of IPA, the coupling of the methyl-carbon and the ethyl-hydrogen is 3,4 Hz in the *cis*-configuration, while it is 8,1 Hz in the *trans-*configuration. The detection of a J_{CCCH} equal to 3,7 Hz confirms for certain that H₁₀ and COOH are in cis-configuration to one another. Hence the IPA configuration is of (Z)-type, as additionally already suggested by the coupling constant J_{H10,OH} being 1,8 Hz in the proton spectra of the first set. In the opposite configuration (E) the coupling constant J_{CCCH} would have had a value of about 8 Hz.
c) The signal at 140 ppm is a doublet (J=2Hz) of all of both ethylene and aromatic quaternary carbons of IPA. Only C₁₁ can have a single coupling, particularly with H₁₀. Consequently, results confirmed the allotment of the 140 Hz signal to C₁₁, made by exclusion in the discussion of the fifth set of spectra.
d) The remaining quaternary signals (C₃, C₈ and C₉) show a plurality consistent with the above results
e) The 129,8 ppm signal shows a direct coupling constant J_{CH} of 187,5 Hz, far larger than all other CH's, whose values range from 157 to 160 Hz, typical of sp² carbons. Hence the 129,8 ppm carbon (differently of the other CH's) should be linked to an electronegative atom, such as the indole nitrogen, thus confirming the previous allotment of C₂.

Exemplary preparations of the process according to the invention (A) are reported hereinafter:

### EXAMPLE 1

### Preparation of the enol tautomer form of 3-indolylpyruvic acid

8.0 g tryptophan and 6.0 ml triethylamine are quickly added to 56 ml methanol, maintained at ice bath temperature. The whitish suspension is left under stirring, checking the temperature.

After about 10 min, 5.59 ml picolilaldehyde (PCA, 2-pyridinecarboxaldehyde) are added, quickly and under stirring, obtaining a clear canary yellow solution. Ice bath temperature and stirring are maintained for other 30 min.

Then, 5.166 gr zinc acetate dihydrate, stored overnight under vacuum and KOH pellets were quickly added, obtaining, after some minutes, a very fine and fluid canary yellow precipitate.

After 30 min, 12.0 ml triethylamine are added, maintaining it in the ice bath and leaving the yellow suspension under stirring for an additional 30 min.

The suspension is transferred, under vigorous stirring and for about 15 min, in 800 ml 1N HCl, preheated at 55°C, accurately washing the reaction flask with additional 20 ml 1N HCl.

A clear yellow-orange solution, and, after about 5 min from the start of the transferring, a noticeable fine and near-crystal yellow-pale gold precipitation increasing with time, are obtained.

After about 30 min from the end of the transferring, and maintaining the stirring, the heating bath is replaced by a circulating cold water bath.

After 30 more minutes, the precipitate is recovered by vacuum filtration and is washed successively with 30 ml cold acid water (x2) and once with cold distilled water.

The precipitate is dried overnight in a vacuum dryer, recovering 5.370 g (about the 67.4%) of indole-3-pyruvic, as a fine and semi-crystal yellow-pale gold powder, with >99.9% purity (HPLC, NMR, IR and UV in agreement).

### EXAMPLE 2

### Preparation of the enol tautomer form of 3-indolylpyruvic acid

8.0 g tryptophan and 6.0 ml triethylamine are quickly added to 56 ml methanol, maintained at ice bath temperature. The whitish suspension is left under stirring, checking the temperature.

After about 10 min, 4.47 ml picolilaldehyde (PCA, 2-pyridinecarboxaldehyde) and 2.73 ml triethylamine are added, quickly, successively and under stirring, obtaining a clear canary yellow solution. Ice bath temperature and stirring are maintained for 30 more minutes.

Then, 5.166 g zinc acetate dihydrate, stored overnight under vacuum and KOH pellets are quickly added. The solution changes to dark orange, and after a few minutes, a very fine and fluid canary yellow precipitate is obtained.

After 30 min, 12.0 ml triethylamine are added, maintaining it in the ice bath and leaving the yellow suspension under stirring for other 30 min.

The suspension is transferred, under vigorous stirring and in about 15 min, into 800 ml 1N HCl, preheated at 55°C, accurately washing the reaction flask with additional 20 ml 1N HCl.

A clear yellow-orange solution is obtained, in which, after about 5 min from the start of the transferring, a noticeable fine and near-crystal yellow-pale gold precipitation appears, increasing with time.

After about 30 min from the end of the transferring, and maintaining the stirring, the heating bath is replaced by a circulating cold water bath.

After further 30 min, the precipitate is removed by vacuum filtration and is washed successively with 30 ml cold acid water (x2) and once with cold distilled water.

The precipitate is dried overnight in a vacuum dryer, recovering 6.500 gr (about the 81.7%) of indole-3-pyruvic, as a fine and semi-crystal yellow-pale gold powder, with >99.9% purity (HPLC, NMR, IR and UV in agreement).

Exemplary preparations of the salts according to B) of the invention are illustrated hereinafter.

### EXAMPLE 3

### Preparation of the sodium salt of the enol tautomer of 3-indolylpyruvic acid from sodium methoxide in methyl alcohol.

300 mg of 3-indolylpyruvic acid in the enol form are dissolved, under stirring and at room temperature, in 10 ml anhydrous methyl alcohol. At complete dissolution, 2.46 ml 0.5M sodium methoxide solution in anhydrous methanol, freshly prepared, and in a quantity such as to neutralize all the acid, are added. After stirring for one hour at room temperature, the reaction mixture is concentrated under vacuum to about one third, and added dropwise to 200ml anhydrous ethyl ether under vigorous stirring, obtaining a very fine yellowish precipitate, while the solution tends to discolor. After about two hours, the precipitate is recovered on sintered glass filter (G4 type) by suction filtering. The precipitate thus recovered is washed twice with 10 ml anhydrous ethyl ether and kept overnight under vacuum on KOH pellets. 272 mg (>98% yield) sodium salt of the enol form of 3-indolylpyruvic acid (>98% purity HPLC in free acid) are recovered.

### EXAMPLE 4

### Preparation of the sodium salt of the enol tautomer of 3-indolylpyruvic acid from sodium ethoxide in ethyl alcohol.

With a procedure similar to that of example 3, the sodium salt of the enol form of 3-indolylpyruvic acid was prepared from sodium ethoxide in anhydrous ethyl alcohol (21% w/w, amount used equal to acid neutralization). Amount of enol tautomer form of 3-indolylpyruvic acid, reaction solvent, reaction yield and purity are identical to those of example 3.

### EXAMPLE

### Preparation of the sodium salt of the enol tautomer of 3-indolylpyruvic acid from sodium methoxide powder.

300 mg enol form of 3-indolylpyruvic acid are suspended in 70 ml anhydrous ethyl ether and kept under stirring at room temperature until complete dissolution, obtaining a deep yellow-orange solution. 70.45 mg sodium methoxide powder (>95% purity), in an amount such as to neutralize all the acid, and 963 µl anhydrous methyl alcohol as reaction initiator are quickly added to this solution, maintaining a vigorous stirring. An abundant fine yellowish precipitation that increases with time is obtained. The reaction mixture is left overnight under stirring at room temperature. The yellowish precipitate thus obtained is recovered on sintered glass filter (G4 type) by suction filtering. The precipitate thus recovered is washed twice with 10 ml anhydrous ethyl ether and kept overnight under vacuum on KOH. 262 mg (>98% yield) sodium salt of the enol form of 3-indolylpyruvic acid (>98% purity HPLC in free acid) are recovered.

### EXAMPLE 6

### Preparation of the sodium salt of the enol tautomer of 3-indolylpyruvic acid from metal sodium.

300 mg enol form of 3-indolylpyruvic acid were suspended in 70 ml anhydrous ethyl ether and kept under stirring and at room temperature until complete dissolution, obtaining a deep yellow-orange solution. To this solution, 34 mg metal sodium cleaned, quickly weighted and in an amount such as to neutralize all the acid, and 500 µl anhydrous methyl alcohol as metal sodium activator were added. A flocculent yellow precipitation that increases with time is obtained. It is left overnight under stirring, and the precipitate is recovered as in example 5. 251 mg (>93% yield) sodium salt of the enol form of 3-indolylpyruvic acid (>98% purity HPLC in free acid) are recovered.

### EXAMPLE 7

With a procedure similar to that of example 5, the following were prepared:
- potassium salt of the enol tautomer of 3-indolylpyruvic acid starting from the potassium methoxide powder;
- lithium salt of the enol tautomer of 3-indolylpyruvic acid starting from the lithium methoxide powder.

Recovery yields and purities of the corresponding salts are similar to those of example 5.

### EXAMPLE 8

With a procedure similar to that of example 3, the lithium salt of the enol tautomer of 3-indolylpyruvic acid was prepared starting from lithium methoxide in anhydrous methanol. Recovery yields and purities of the corresponding lithium salt are similar to those of example 3.

### EXAMPLE 9

With a procedure similar to that of example 6, the potassium salt of the enol tautomer of 3-indolylpyruvic acid was prepared, starting from the metal potassium activated *in situ* with anhydrous methyl alcohol. Recovery yields and purities of the corresponding potassium salt are similar to those of example 6.

### EXAMPLE 10

### Preparation of the magnesium salt of the enol tautomer of 3-indolylpyruvic acid from metal magnesium.

300 mg enol form of 3-indolylpyruvic acid are suspended in 70 ml anhydrous ethyl ether and kept under stirring at room temperature until complete dissolution, obtaining a deep yellow-orange solution. To this solution 17.9 mg metal magnesium, cleaned, quickly weighted and in a quantity such as to neutralize all the acid, and 500 µL anhydrous methyl alcohol as metal sodium activator are added. There ensues a flocculent yellow precipitation increasing with time. It is left overnight under stirring, and the precipitate is recovered as in example 5. 300 mg (>95% yield) magnesium salt of the enol form of 3-indolylpyruvic acid (>98% purity HPLC in free acid) are recovered.

### EXAMPLE 11

### Preparation of the magnesium salt of the enol tautomer of 3-indolylpyruvic acid from magnesium methoxide.

300 mg of the enol form of 3-indolylpyruvic acid were suspended in 70 ml anhydrous ethyl ether and kept under stirring at room temperature until complete dissolution, obtaining a deep yellow-orange solution. To this solution, maintaining a vigorous stirring, 1.18 ml magnesium methoxide in anhydrous methyl alcohol solution (6% w/w concentration) were quickly added, in an amount such as to neutralize all the acid. An abundant fine yellowish precipitation that increases with time is obtained. The reaction mixture is left for three hours under stirring and at room temperature. The very fine yellowish precipitate thus obtained is recovered by filtration on a paper filter (Wathman No.42, washed with anhydrous ethyl ether and brought to weight constancy) under light suction. The precipitate, thus recovered on paper filter, is washed twice with 10 ml anhydrous ethyl ether and kept overnight under vacuum on KOH pellets. 282 mg (>98% yield) magnesium salt of the enol form of 3-indolylpyruvic acid (>98% purity HPLC in free acid) are recovered.

### EXAMPLE 12

### Preparation of the calcium salt of the enol tautomer of 3-indolylpyruvic acid from calcium methoxide powder

With a procedure similar to that of example 5, the calcium salt of the enol form of 3-indolylpyruvic acid was prepared, starting from calcium methoxide powder. Recovery yields and purity of the corresponding calcium salt are similar to those of example 11.

### 2. Identification of the enol form of 3-indolylpyruvic acid in solution

The enol form of 3-indolylpyruvic acid, obtained at a >99.9% purity with the method reported above in examples 1 and 2, appears wholly insoluble in water and in chloroform, slightly soluble in ethyl ether, and highly soluble in acetone, methanol and ethanol. The solution in methanol exhibits, under UV light, the characteristic peak at 325 nm, allowing to distinguish the tautomer from the keto form, in which the peak disappears and instead the 280 nm peak of the indole ring predominates. The enol form of 3-indolylpyruvic acid appears extremely stable in methanol, and even after several days at room temperature no relevant losses are detected.

When the enol tautomer of 3-indolylpyruvic acid is solubilized in an aqueous environment, the decline of the UV peak at 325 nm, more or less quick according to the solution temperature, the pH and the type of buffer used is immediately observed. In the light of the published works, this demonstrates the establishing of the well-known keto-enol equilibrium, which in aqueous solutions largely favors the keto tautomer when the aqueous solutions have a neutral or basic pH. In the experimental conditions used, a quicker disappearance of the 325 nm peak is observed when the enol tautomer of the ketoacid is solubilized in diluted NaOH solutions; it follows that in this solvent the keto-enol equilibrium shifts towards the keto form in a few seconds.

However, contrarily to what described in the previous literature, the keto form in the aqueous solutions is no more than minimally capable of reconstituting the starting enol form (characterized by the 325 nm peak in UV), unless extremely drastic conditions are used, such as the addition of concentrated acid solutions; in this case as well, the 325 nm peak appears extremely unstable. Therefore, the studies carried out with the near-pure form of the enol tautomer indicate that in alkali or neutral aqueous solutions rather than an actual keto-enol equilibrium there occurs a more or less quick transformation of the enol form in the keto form, without appreciable keto-to-enol reversion.

More accurate studies, obtained eluting the solutions in high pressure liquid chromatography (HPLC with Bondapack C18 column) and revealing the indoles, allowed to confirm the more or less rapid disappearance of the enol form in the neutral or basic aqueous solutions, accompanied by the increase, already in short times, of a new chromatographic peak characterized by the keto form of its gem-diole and not by that of the ketoacid. At longer incubation times also the gem-diole form disappears, without giving rise to the keto form.

In particular, using a phosphate solution at pH 7.4, it is observed that the enol form drops to the 44% already at +30 min, whereas the gem-diole form begins to predominate (increase) at +1 hour (44% *vs* 23%), yet it stabilizes at about +2 hours (49%), and then gradually declines.

In short, the tests on the keto-enol equilibrium of the near-pure form of the enol tautomer of 3-indolylpyruvic acid lead to conclude that the enol tautomer, upon having been solubilized in neutral or alkali aqueous solutions, may not anymore be reconstituted in relevant concentrations from the keto tautomer form, and that the keto tautomer is quickly shifted towards the gem-diole form, which appears unstable and progresses to further chemical transformations. This entails important practical consequences, as the 3-indolylpyruvic acid will exhibit the properties of the enol form or those of the gem-diole form, depending on the solubilization conditions used. Moreover, once the equilibrium has been shifted towards the gem-diole form, the chemico-physical, physiological and pharmacological properties of the enol form will eventually be lost.

### 3. Identification of the enol tautomer form of 3-indolylpyruvic acid in salines

The near-pure enol tautomer of 3-indolylpyruvic acid, obtained by the method described above in examples 1 and 2, was investigated on rat tissue homogenates, in order to highlight its participation in the methabolic pathways characterizing the use of tryptophan and of its derivatives in mammalian cells. In particular, since the experiments in aqueous solutions (see 2) ) demonstrated that the enol form of the ketoacid quickly disappears at neutral or alkaline pH, the behavior of the enol form (obtained dissolving the ketoacid, obtained as described in examples 1 and 2, immediately prior to the experiments) was analyzed in comparison to the keto form (obtained dissolving the ketoacid purchased from commercial sources in diluted NaOH) in the two main pathways accessible to the 3-indolylpyruvic acid in mammalian cells: the transformation into Tryptophan (catalyzed by different transaminases) and that into 3-indolyl-lactic acid, catalyzed by specific dehydrogenases. Using the keto form no significant transformations to tryptophan, nor to 3-indolyl-lactic acid was observed, whereas the results obtained with the enol form highlighted the extraordinary capability of this chemical species of acting as precursor to both indole compounds, and of maintaining a good stability also in slightly alkaline buffer environments (pH 7.4), without shifts toward the gem-diole form observed in the mere aqueous solutions devoid of biological material. This phenomenon, ascribed to the presence in biological fluids of a specific tautomerase already reported in literature, allows the enol form to be subjected to biochemical transformations by specific enzymes active on the indoles. In particular, when the enol form of the ketoacid is incubated in rat liver homogenates, a gradual transformation of the enol of 3-indolylpyruvic acid in tryptophan is observed: the transformation is rather linear in time, is independent from the pH - at least in the 6.5÷7.5 interval - and it leads to the conversion of over the 50% of the ketoacid in 5 hours. The only other indole identified in the reaction is 3-indolealdehyde, whose concentration however is modest and does not change with time. No transformation into 3-indolyl-lactic acid was observed in liver homogenates.

Conversely, when the enol of 3-indolylpyruvic acid is incubated in rat brain homogenate, the bulk of transformation occurs towards the 3-indolyl-lactic acid pathway, whereas more modest amounts are converted to tryptophan, and small amounts are found as 3-indolealdehyde, whose concentration substantially does not change over time. The conversion in brain tissue appears quicker, and in two hours about the 50% of the ketoacid is found as 3-indolyl-lactic acid. In short, the experiments carried out with the two tautomer forms of 3-indolylpyruvic acid on mammalian organ homogenates allow to state that the enol form, unstable in mere aqueous solvents at physiological pH, is stabilized in tissues, by the intervention of one or more tautomerases, and it represents the substrate used by cells both for the pathway leading to tryptophan and for that leading to indolyllactic acid. Moreover, the discovery that the 3-indolyl-lactic acid appears as the predominant metabolite in brain after incubation of the enol tautomer of 3-indolylpyruvic acid suggests that the 3-indolyl-lactic acid actually serves as a reservoir, from which the indole alpha-ketoacid may, when required,be obtained, and which may therefore be used in lieu of the ketoacid for some of the pharmacological effects carried out thereby on the Central Nervous System.

### 4. Absorption of the enol tautomer form of 3-indolylpyruvic acid

Since the experiments on rat organ homogenates highlighted the peculiar capability by the enol form of 3-indolylpyruvic acid of remaining stable, by tautomerase intervention, over sufficiently long times, studies were carried out for determining ketoacid levels after administration to animals via oral route. Due to the acid environment of the stomach (known to favor enol form stability) and to the presence of tautomerase in tissues, the enol form of the ketoacid was demonstrated to be well absorbed and to give plasma levels which, exhibiting a peak between +1 and +2 hours, are still clearly identifiable after several hours.

For instance, when the enol form of 3-indolylpyruvic acid was acutely ¹ administered by gastric lavage to rats at a dose of 100 mg/Kg, at +1 hour 74 ± 14 micrograms(mcg)/ml of plasma were found, whereas rats chronically treated with the same dose exhibited plasma levels equal to 183 ± 78 mcg/ml. Raising the dose to 400 mg/Kg, values of 160 ± 13 mcg/ml for acute administration and of 189 ± 68 mcg/ml for chronic administration were obtained.

In dogs as well analogous results were obtained, as the administration (in food-mixed pellets) of 100 mg/Kg enol form of 3-indolylpyruvic acid led, at +1 hour, to plasma rates of 106 ± 47 mcg/ml in acute, and of 97 ± 17 mcg/ml in chronic. 285 mg/Kg doses produced, at +1 hour, concentrations of 136 ± 17 mcg/ml in acute, and of 120 ± 8 mcg/ml in chronic.

Hence, these results allow to conclude that the enol form of 3-indolylpyruvic acid is well-absorbed at a gastric level, and is stabilized under the conditions predominating in biological environments, so as to be still detectable in plasma even after several hours.

### 5. Kynurenic acid formation

It is already known from literature that the 3-indolylpyruvic acid may function, in suitable methabolic situations, as direct precursor to the kynurenic acid, the most important endogenous inhibitor of the excitatory amino acids. It has also been described (Advances Exptl.Med.Biol. 294, 515-18, 1991) that the enol form of the ketoacid is much more effective than the keto one in capturing the free radicals of Oxygen that facilitate the conversion to kynurenic acid. Therefore, comparison tests were conducted between the enol form of 3-indolylpyruvic acid, obtained according to the method described above in examples 1 and 2, the keto form (obtained solubilizing a standard preparation of 3-indolylpyruvic acid obtained from commercial sources in 0.1M NaOH), and a mixture of the two forms (obtained solubilizing the above standard preparation in a pH 7.4 phosphate buffer). Thus, it has first of all been found that, contrarily to the keto tautomer form, the enol form of 3-indolylpyruvic acid has a strong neutralizing power, not merely towards the superoxide anion, but also towards the hydroxyl ions, which are generally considered to be the most dangerous in cell degeneration phenomena. Moreover, the enol form is found to be 100 to 1000 times more powerful than the keto one, both in protecting membrane phospholipids from degradation and in blocking luminol chemiluminescence induced by a xanthin/xanthin oxidase mixture. Concerning the formation of kynurenic acid, under the same experimental conditions the enol form of 3-indolylpyruvic acid always produced from 20 to 100 times more kynurenic acid than the keto form or the mixture of the two forms. When the enol form of 3-indolylpyruvic acid was injected intravenously (200 mg/Kg) in baboons, the enol plasma concentration curve gradually dropped from the high initial values, remaining at clearly detectable levels for a few hours. Concomitantly, kynurenic acid began to form, reaching a plasma peak at +90-+180 min from the enol injection, with remarkable interindividual variations. Kinetic curves showed that that at least 2 to 10% of the enol form of 3-indolylpyruvic acid was transformed in kynurenic acid.

The results of these tests confirm that the 3-indolylpyruvic acid in enol form is the main precursor to the kynurenic acid, and that this transformation in mammalian biological fluids *in vivo* takes place in a consistent and continuous manner, so that the enol may actually be considered as a reservoir, capable of continuously releasing kynurenic acid in the blood stream for several hours.

### 6. Pharmacological activities

The enol form of 3-indolylpyruvic acid, obtained with the methods hereto described in examples 1 and 2, was subjected to several pharmacological tests, in order to define its potential therapeutic activities, taking into account the capability of the enol form of acting as tryptophan precursor (as demonstrated in the experiments on mammalian tissue homogenates), as well as the fact that the compound may increse cerebral levels of indolyl-lactic acid, and continuously produces relevant amounts of kynurenic acid, reckoned to be a powerful inhibitor of excitatory amino acids. The tests, carried out in comparison both to the keto form of 3-indolylpyruvic acid (obtained solubilizing a standard preparation of 3-indolylpyruvic acid obtained from commercial sources in 0.1 M NaOH) and to a mixture of the two tautomers (obtained solubilizing the above standard preparation in a pH 7.4 phosphate buffer), enabled to ascertain that the enol tautomer of 3-indolylpyruvic acid, administered to rats both orally and by IP injection, possesses several pharmacological properties evidenced both on the central nervous system (e.g. prolongation of sleep, central analgesia, protection from NMDA-induced convulsions, reduction of toxic effects from opiates, anxiolytic and antidepressant effects, stress inhibition) and on the cardiovasculary system (pressure reduction in hypertensive rats, but not in normotensive rats). The observed effects were clearly superior to those obtained with the mixture of the two tautomers, whereas the keto tautomer *per se* exhibited no apparent effects on the considered parameters.

Concerning the salts of the enol tautomer form of 3-indolylpyruvic acid, obtained according to the methods reported in examples 3 to 12, they were assayed in two models of human cell cultures, melanoma and neuroblastoma, in order to determine their specific pharmacological activities.

The results obtained are reported hereinafter:

### EXAMPLE 13

### Effect of the enol tautomer of 3-indolylpyruvic acid and of its salts on melanoma cell invasion through Matrigel

Matrigel is an extracellular biological matrix extracted from the Englebreth-Holm-Swarm murine sarcoma, composed of the major components of the basement membrane like laminin, entactin, collagen IV and proteoglycans. At room temperature, Matrigel forms a gel that can promote the growth of primary and continuous cell lines, and moreover can act as medium (substrate) for the adhesion, the differentiation, the migration and the invasion of tumor cell lines. Due to such features, a Matrigel test is commonly used in order to characterize the activity of substances capable of interfering with the motility of tumor cells and of inflammatory response cells. The test is conducted in wells partitioned by an 8 micron pore size membrane: Matrigel (12.4 micrograms) is uniformly spread on the membrane, whereas a chemoattractant (600 microliters), consisting of the supernatant of a murine hembryo cell line (3T3) is inserted in the bottom section of the well. The cells to be tested are seeded in the top section of the well, whereas the compounds that could interfere with their motility are placed above as well as below the membrane, so as to prevent the formation of a concentration gradient. At the end of the experiment, the cells that have crossed the membrane are counted.

Human melanoma cells (A2058 line) were cultured in MEM medium, in a 5% CO2 at 37° C. Having reached the desired growth level, the (100.000) cells were seeded in the top section of the well. The enol of 3-indolylpyruvic acid and its salts, solubilized in the cell culture medium were placed in the top and bottom section of the well.

**Results:**

| **Compound** | **Concentration (**µ**M)** | **Inhibition %** |
|---|---|---|
| 3-tea ENOL | 500 | 97 ± 2 |
| 3-IPA ENOL | 250 | 77 ± 11 |
| 3-IPA ENOL | 125 | 44 ± 5 |
| Na salt | 250 | 78 ± 3 |
| Na salt | 125 | 52 ± 1 |
| K salt | 250 | 78 ± 3 |
| K salt | 125 | 51 ± 3 |
| Ca salt | 140 | 82 ± 6 |
| Ca salt | 70 | 38:L 10 |
| Li salt | 250 | 91 ± 5 |
| Li salt | 125 | 48 ± 7 |
| Mg salt | 130 | 66 ± 7 |
| Mg salt | 65 | 46 ± 14 |

The values relate to the average of three distinct experiments, ± is the Standard Deviation.

As it is apparent from the above Table, both the enol tautomer of 3-indolylpyruvic acid and its salts exhibit relevant inhibitory effects on the motility of human melanoma cells through Matrigel. This effect, connected to the discovery that the antagonists of excitatory amino acids may slow down tumor growth (Nature Medicine, 7, 1010-1015, 2001), suggests that the enol tautomer, and all the more some of its salts, could be usefully adopted in tumor treatment.

### EXAMPLE 14

### Effect of the enol tautomer of 3-indolylpyruvic acid and of its derivatives on neuronal cell sprouting.

Neurons are cells extraordinarily capable of sprouting elongations (neurites), underlying the complex interconnections of the Central Nervous System, and which are generally viewed as a cell health index: in fact, it is known that the neurons of younger individuals are characterized by several and long neurites, whereas the neurons of elder individuals or of persons affected by Central Nervous System degenerative diseases exhibit cerebral areas in which the neurons have few neurites, and of limited length. It is also known that neuronal tumor cells in general grow with short and few neurites, though, when subjected to the action of trophic factors, they can arrest growth and start developing longer and more numerous neurites.

LANS human neuroblastoma cells were seeded in 6-well plates (25.000 cells/well) in DMEM (Dulbecco's Modified Eagle's Medium) medium, additioned with 10% fetal serum and 40 micrograms/ml gentamycin. After 24 hours, the enol tautomer of 3-indolylpyruvic acid or its derivatives, at an end concentration of 10 µM was added to the culture medium. The treatment was continued for 7 days, with medium replacement and addition of the compound under test every three days. At +7 days the cells were photopgraphed, using a Leitz Dialux 20 microscope. Then, neurite length was measured.

The results, reported in the following Table, express, in microns, the average length of the neurites for each treatment; ± is the standard deviation:

| **TREATMENT** | **VALUE (micrometers)** |
|---|---|
| Untreated | 21.8 ± 8.8 |
| 3-IPA Enol | 31.9 ± 6.8 |
| 3-Indolyllactic acid | 39.7 ± 11.8 |
| 3-Indolylacetic acid | 23.3 ± 10.5 |
| 5-Hydroxy- Indolylacetic acid | 25.7 ± 13.2 |
| Enol Na salt | 28.3 ± 16.4 |
| Enol K salt | 44.1 ± 12.1 |
| Enol L salt | 29.4 ± 8.5 |
| Enol Mg salt | 40.1 ± 17.5 |
| Enol Ca salt | 32.7 ± 8.3 |

The results are of great interest, and show that the enol tautomer of 3-indolylpyruvic acid and some of its derivatives are capable of stimulating the neurons of a human tumor cell line to sprout neurites, which generally are a symptom of differentiation and vitality. These effects may be partly due to the capability exhibited by antagonists of the excitatory amino acids of reducing the growth of neuronal tumors (Nature Medicine, 7, 1010-1015, 2001), yet they are mainly due to the remarkable antioxidizing effects shown by the enol tautomer of 3-indolylpyruvic acid. It should be stressed that, among the assayed indoles, only the indolelactic acid exhibits noticeable effects on neurite length, even superior to those of the enol form, (probably since it acts as reservoir, continually supplying a compound having antioxidizing activity), whereas the 3-indoleacetic acid and the 5-hydroxy-indoleacetic acid, whose antioxidizing power is very limited, do not influence neurite length under the experimental conditions used.

### 7. Formulations

Due to its instability in aqueous solutions, the enol tautomer of 3-indolylpyruvic acid cannot be formulated in pharmaceutical form with the common water-using technological systems, and specific protection systems shall be resorted to. Acceptable forms may be capsules and tablets for oral use (in this latter case with technological contrivances, for the manufacturing as well as for the coating and the optional presence of absorption retarding agents), and powders to be solubilized immediately prior to use, for injectable products, using any one of the salts described in the invention.

The dosage envisaged for human use ranges from 100 to 500 mg per dose.

## Claims

1. Enol tautomeric form of 3-indolylpyruvic acid in a chemically stable, semicrystalline, solid state, having enol purity not lower than 99% referred to the total acid.

2. Enol tautomeric form of 3-indolyl-pyruvic acid according to claim 1, having an enol purity of 99 to 99,9% to the total acid.

3. Enol tautomeric form of 3-indolyl-pyruvic acid according to claim 1 or 2, which consists of the (Z) form of the geometric isomer thereof, in a chemically and shelf life stable semicrystalline solid state.

4. Enol tautomeric form of 3-indolyl-pyruvic acid according to any one of claims 1 to 3, wherein said semicrystalline solid state comprises a needle-shaped crystal form.

5. Enol tautomeric form of 3-indolyl-pyruvic acid, according to any one of claims 1 to 4 for use as a medicament.

6. Enol tautomeric form of 3-indolyl-pyruvic acid, according to claim 5 for use in a therapeutic treatment of disturbances of the central nervous system.

7. Enol tautomeric form of 3-indolyl-pyruvic acid, according to claim 5 or 6, for use in a treatment of anxiety, depression, sleep disturbances, appetite disturbances, mood and emotionality disturbances, cerebral post-trauma disturbances, cerebral hypoxia and ischemia, epilepsies, elder cognition declination, neurodegenerative disturbances, physical and psychological stress situations.

8. Enol tautomeric form of 3-indolyl-pyruvic acid, according to claim 5 for use in a treatment of cardiovascular pathologic situations.

9. Enol tautomeric form of 3-indolyl-pyruvic acid, according to claim 5 for use in a therapeutic treatment for the sprouting of neuron cells.

10. Enol tautomeric form of 3-indolyl-pyruvic acid, according to claim 5 for use in a therapeutic treatment of tumors.

11. Enol tautomeric form of 3-indolyl-pyruvic acid, according to any one of claims 1 to 4 for use in the pharmaceutical industry as a chemically pure and shelf stable material for the manufacture of drugs.

12. A process for the production of the enol tautomeric form of 3-indolyl-pyruvic acid according to any one of claims 1 to 4, comprising reacting the amino function of tryptophan with an aromatic aldehyde, in the presence of a complexant agent and a proton acceptor amine base, in a reaction solvent and hydrolyzing the product of said coupling reaction in a hot acidic hydrolysis step at a temperature of 45 to 65 °C, wherein
the aromatic aldehyde is 2-pyridine-carboxyaldehyde alone or in a mixture with triethylamine,
the proton acceptor non-nucleophilic amine base is triethylamine,
the complexant agent is selected from the group consisting of zinc acetate or zinc sulfate,
and the organic polar solvent as a reaction solvent is selected in the group consisting oftetrahydrofuran, dimethylformamide, acetonitrile, and methanol.

13. A process according to claim 12, wherein said complexant agent is Zn acetate bihydrate, or Zn sulfate heptahydrate.

14. A process according to claim 12 or 13, wherein the temperature of said hot acidic hydrolysis step is 55 °C.

15. A process according to any one of claims 12, 13 or 14, wherein said reaction solvent is not anhydrous.

16. A salt with an alkaline or earth alkaline metal of the enol tautomeric form of 3-indolyl-pyruvic acid according to any one of claims 1 to 4 in a pure, stable and crystal form.

17. The salt according to claim 16, wherein said alkaline or earth-alkaline metal is sodium, lithium, potassium, magnesium or calcium.

18. The salt of the enol tautomeric form of 3-indolyl-pyruvic acid according to claim 17 for use as a medicament.

19. The salt of the enol tautomeric form of 3-indolyl-pyruvic acid according to claim 18, for use in the therapeutic treatment of disturbances of the central nervous system.

20. The salt according to claim 19 wherein said disturbances are anxiety, depression, sleep disturbances, appetite disturbances, mood and emotionality disturbances, cerebral post-trauma disturbances, cerebral hypoxia and ischemia, epilepsy, elder cognition declination, neurodegenerative disturbances, physical and psychological stress situations.

21. The salt of the enol tautomeric form of 3-indolyl-pyruvic acid according to claim 18, for use in a therapeutic treatment of pathologic cardiovascular situations.

22. The salt of the enol tautomeric form of 3-indolyl-pyruvic acid according to claim 18, for use as an active agent for a therapeutic treatment of a deficiency of serotoninergic activity or an excess of excitatory amino acid activity.

23. The salt according to claim 18, for use as an active agent in a therapeutic treatment for the sprouting of neuron cells.

24. The salt according to claim 18, for use as an active agent in a therapeutic treatment of tumors.

25. A process for the production of an alkaline or earth-alkaline metal salt according to claim 16 or 17 of the enol tautomeric form of 3-indolyl-pyruvic acid **characterized by** the step of neutralizing said enol tautomer of 3-indoly-pyruvic acid with an alkaline or earth-alkaline metal, in a first aprotic polar solvent, together with a second protic polar solvent as a metal activator.

26. A process according to claim 25, wherein said first aprotic and said second protic polar solvent are anhydrous.

27. A process according to claim 25 or 26, wherein said aprotic polar solvent is diethyl-ether or tetrahydrofuran.

28. A process according to any one of claims 25 to 27, wherein said neutral metals are under form of methoxides and ethoxides and said protic solvent is methyl-alcohol or ethyl-alcohol.

29. A process according to any one of claims 25 to 28, wherein said alkaline or earth-alkaline metal is sodium, lithium, potassium, magnesium or calcium.

30. Enol tautomeric form of 3-indolyl-pyruvic acid according to any one of claims 1 to 4 or an alkaline or earth-alkaline salt thereof according to claim 16 or 17, for use as a precursor of 3-indolyl-lactic acid, for a therapeutic treatment of patients in need of 3-indolyl-lactic acid administration.

31. Enol tautomeric form of 3-indolyl-pyruvic acid according to any one of claims 1 to 4 or an alkaline or earth alkaline salt thereof according to claim 16 or 17, for use as a precursor of kynurenic acid, for a therapeutic treatment of patients in need of kynurenic acid adminisration.

32. Pharmaceutical composition comprising the enol tautomeric form of 3-indolyl-pyruvic acid, according to any one of claims 1 to 4 or a salt thereof with an alkaline or earth-alkaline metal according to any one of claims 16 or 17, as well as a pharmaceutically compatible excipient or carrier.

33. Pharmaceutical composition according to claim 32, wherein said carrier is a physiologic solution.

34. Pharmaceutical composition according to claim 32 or 33 for use in a therapeutic treatment of the central nervous system, pathologic cardiovascular situations, neuron sprouting and tumors.

35. Pharmaceutical composition according to claim 32 or 33 for use in a treatment of anxiety, depression, sleep disturbances, appetite disturbances, mood and emotionality disturbances, cerebral post-trauma disturbances, cerebral hypoxia and ischemia, epilepsies, elder cognition declination, neurodegenerative disturbances, physical and psychological stress situations.

36. Pharmaceutical composition according to claim 32 or 33, in the form of capsules, tablets, suppositories or injectable vials.

## Patentansprüche

1. Enol-tautomere Form der 3-Indolylbrenztraubensäure in einem chemisch stabilen, halbkristallinen festen Zustand mit einer Enol-Reinheit von nicht geringer als 99%, bezogen auf die gesamte Säure.

2. Enol-tautomere Form der 3-Indolylbrenztraubensäure gemäß Anspruch 1 mit einer Enol-Reinheit von 99 bis 99,9 %, bezogen auf die gesamte Säure.

3. Enol-tautomere Form der 3-Indolylbrenztraubensäure gemäß Anspruch 1 oder 2, welche aus der (Z)-Form des geometrischen Isomers davon in einem chemisch- und lager-stabilen halbkristallinen festen Zustand besteht.

4. Enol-tautomere Form der 3-Indolylbrenztraubensäure gemäß einem der Ansprüche 1 bis 3, wobei der halbkristalline feste Zustand eine nadelförmige kristalline Form umfasst.

5. Enol-tautomere Form der 3-Indolylbrenztraubensäure gemäß einem der Ansprüche 1 bis 4 zur Verwendung als ein Medikament.

6. Enol-tautomere Form der 3-Indolylbrenztraubensäure gemäß Anspruch 5 zur Verwendung in einer therapeutischen Behandlung von Störungen des zentralen Nervensystems.

7. Enol-tautomere Form der 3-Indolylbrenztraubensäure gemäß Anspruch 5 oder 6 zur Verwendung in einer Behandlung von Angst, Depression, Schlafstörungen, Appetitstörungen, Stimmungs- und Emotionsschwankungen, cerebralen posttraumatischen Störungen, cerebraler Hypoxie und Ischämie, Epilepsien, durch das Alter bedingte Wahrnehmungsstörungen, neurodegenerativen Störungen, physischen und psychischen Stresssituationen.

8. Enol-tautomere Form der 3-Indolylbrenztraubensäure gemäß Anspruch 5 zur Verwendung in einer Behandlung von cardiovaskulären pathologischen Situationen.

9. Enol-tautomere Form der 3-Indolylbrenztraubensäure gemäß Anspruch 5 zur Verwendung in einer therapeutischen Behandlung zum Wachsen (sprouting) von Neuronenzellen.

10. Enol-tautomere Form der 3-Indolylbrenztraubensäure gemäß Anspruch 5 zur Verwendung in einer therapeutischen Behandlung von Tumoren.

11. Enol-tautomere Form der 3-Indolylbrenztraubensäure gemäß einem der Ansprüche 1 bis 4 zur Verwendung in der Arzneimittelindustrie als ein chemisch reines und lagerstabiles Material zur Herstellung von Medikamenten.

12. Verfahren zur Herstellung der Enol-tautomeren Form der 3-Indolylbrenztraubensäure gemäß einem der Ansprüche 1 bis 4, umfassend das Umsetzen der Aminofunktion von Tryptophan mit einem aromatischen Aldehyd in Gegenwart eines Komplex bildenden Mittels und einer Aminobase als Protonenakzeptor in einem Reaktionslösungsmittel, und Hydrolisieren des Produkts der Kupplungsreaktion in einem heißen Säurehydrolyseschritt bei einer Temperatur von 45 bis 65°C, wobei
das aromatische Aldehyd 2-Pyridincarboxyaldehyd allein oder in einem Gemisch mit Triethylamin ist,
die nicht nukleophile Aminobase als Protonenakzeptor Triethylamin ist,
das Komplex bildende Mittel ausgewählt ist aus Zinkacetat oder Zinksulfat,
und das organische polare Lösungsmittel als ein Reaktionslösungsmittel ausgewählt ist aus Tetrahydrofuran, Dimethylformamid, Acetonitril und Methanol.

13. Verfahren gemäß Anspruch 12, wobei das Komplex bildende Mittel Zn-Actetatdihydrat oder Zn-Sulfatheptahydrat ist.

14. Verfahren gemäß Anspruch 12 oder 13, wobei die Temperatur des heißen Säurehydrolyseschritts 55°C beträgt.

15. Verfahren gemäß einem der Ansprüche 12, 13 oder 14, wobei das Reaktionslösungsmittel nicht wasserfrei ist.

16. Salz mit einem Alkali- oder Erdalkalimetall der Enol-tautomeren Form der 3-Indolylbrenztraubensäure gemäß einem der Ansprüche 1 bis 4 in einer reinen, stabilen und kristallinen Form.

17. Salz gemäß Anspruch 16, wobei das Alkali- oder Erdalkalimetall Natrium, Lithium, Kalium, Magnesium oder Calcium ist.

18. Salz der Enol-tautomeren Form der 3-Indolylbrenztraubensäure gemäß Anspruch 17 zur Verwendung als ein Medikament.

19. Salz der Enol-tautomeren Form der 3-Indolylbrenztraubensäure gemäß Anspruch 18 zur Verwendung in der therapeutischen Behandlung von Störungen des zentralen Nervensystems.

20. Salz gemäß Anspruch 19, wobei die Störungen Angst, Depression, Schlafstörungen, Appetitstörungen, Stimmungs- und Emotionsschwankungen, cerebrale posttraumatische Störungen, cerebrale Hypoxie und Ischämie, Epilepsie, durch das Alter bedingte Wahrnehmungsstörungen, neurodegenerative Störungen, physische und psychische Stresssituationen sind.

21. Salz der Enol-tautomeren Form der 3-Indolylbrenztraubensäure gemäß Anspruch 18 zur Verwendung in einer therapeutischen Behandlung von pathologischen cardiovaskulären Situationen.

22. Salz der Enol-tautomeren Form der 3-Indolylbrenztraubensäure gemäß Anspruch 18 zur Verwendung als ein Wirkstoff in einer therapeutischen Behandlung einer Einschränkung der Serotoninaktivität oder eines Überschusses anregender Aminosäureaktivität.

23. Salz gemäß Anspruch 18 zur Verwendung als ein Wirkstoff in einer therapeutischen Behandlung zum Wachsen (sprouting) von Neuronenzellen.

24. Salz gemäß Anspruch 18 zur Verwendung als ein Wirkstoff in einer therapeutischen Behandlung von Tumoren.

25. Verfahren zur Herstellung eines Alkali- oder Erdalkalimetallsalzes gemäß Anspruch 16 oder 17 der Enol-tautomeren Form der 3-Indolylbrenztraubensäure, **gekennzeichnet durch** den Schritt des Neutralisierens des Enol-Tautomers der 3-Indolylbrenztraubensäure mit einem Alkali- oder Erdalkalimetall in einem ersten aprotischen polaren Lösungsmittel, zusammen mit einem zweiten protischen polaren Lösungsmittel als ein Metallaktivator.

26. Verfahren gemäß Anspruch 25, wobei das erste aprotische und das zweite protische polare Lösungsmittel wasserfrei sind.

27. Verfahren gemäß Anspruch 25 oder 26, wobei das aprotische polare Lösungsmittel Diethylether oder Tetrahydrofuran ist.

28. Verfahren gemäß einem der Ansprüche 25 bis 27, wobei die neutralen Metalle in Form von Methoxiden und Ethoxiden vorliegen und das protische Lösungsmittel Methylalkohol oder Ethylalkohol ist.

29. Verfahren gemäß einem der Ansprüche 25 bis 28, wobei das Alkali- oder Erdalkalimetall Natrium, Lithium, Kalium, Magnesium oder Calcium ist.

30. Enol-tautomere Form der 3-Indolylbrenztraubensäure gemäß einem der Ansprüche 1 bis 4 oder ein Alkali- oder Erdalkalisalz davon gemäß Anspruch 16 oder 17, zur Verwendung als einen Vorläufer der 3-Indolylmilchsäure in einer therapeutischen Behandlung von Patienten, welche die Verabreichung von 3-Indolylmilchsäure benötigen.

31. Enol-tautomere Form der 3-Indolylbrenztraubensäure gemäß einem der Ansprüche 1 bis 4 oder ein Alkali- oder Erdalkalisalz davon gemäß Anspruch 16 oder 17, zur Verwendung als einen Vorläufer der Kynurensäure in einer therapeutischen Behandlung von Patienten, welche die Verabreichung von Kynurensäure benötigen.

32. Arzneimittel, umfassend die Enol-tautomere Form der 3-Indolylbrenztraubensäure gemäß einem der Ansprüche 1 bis 4, oder ein Salz davon mit einem Alkali- oder Erdalkalimetall gemäß einem der Ansprüche 16 oder 17, sowie einen pharmazeutisch verträglichen Exzipienten oder Träger.

33. Arzneimittel gemäß Anspruch 32, wobei der Träger eine physiologische Lösung ist.

34. Arzneimittel gemäß Anspruch 32 oder 33 zur Verwendung in einer therapeutischen Behandlung des zentralen Nervensystems, von pathologischen cardiovaskulären Situationen, Neuronenwachstum (neuron sprouting) und Tumoren.

35. Arzneimittel gemäß Anspruch 32 oder 33 zur Verwendung in einer Behandlung von Angst, Depression, Schlafstörungen, Appetitstörungen, Stimmungs- und Emotionsschwankungen, cerebralen posttraumatischen Störungen, cerebraler Hypoxie und Ischämie, Epilepsien, durch das Alter bedingte Wahrnehmungsstörungen, neurodegenerativen Störungen, physischen und psychischen Stresssituationen.

36. Arzneimittel gemäß Anspruch 32 oder 33 in Form von Kapseln, Tabletten, Zäpfchen oder inj ezierbaren Ampullen.

## Revendications

1. Forme tautomère énolique de l'acide 3-indolylpyruvique dans un état chimiquement stable, semi-cristallin, et solide, ayant une pureté de l'énol pas inférieure à 99 % par rapport à l'acide total.

2. Forme tautomère énolique de l'acide 3-indolyl-pyruvique selon la revendication 1, ayant une pureté de l'énol de 99 à 99,9 % par rapport à l'acide total.

3. Forme tautomère énolique de l'acide 3-indolyl-pyruvique selon la revendication 1 ou 2, qui est constituée par la forme (Z) de son isomère géométrique, dans un état semi-cristallin et solide présentant une stabilité chimique et de conservation.

4. Forme tautomère énolique de l'acide 3-indolyl-pyruvique selon l'une quelconque des revendications 1 à 3, dans lequel l'état semi-cristallin et solide comprend une forme de cristaux aciformes.

5. Forme tautomère énolique de l'acide 3-indolyl-pyruvique selon l'une quelconque des revendications 1 à 4, destinée à être utilisée en tant qu'un médicament.

6. Forme tautomère énolique de l'acide 3-indolyl-pyruvique selon la revendication 5, destinée à être utilisée dans un traitement thérapeutique de troubles du système nerveux central.

7. Forme tautomère énolique de l'acide 3-indolyl-pyruvique selon la revendication 5 ou 6, destinée à être utilisée dans le traitement de l'anxiété, de la dépression, des troubles du sommeil, des troubles de l'appétit, des troubles de l'humeur et de l'émotivité, des troubles cérébraux post-traumatiques, de l'hypoxie et de l'ischémie cérébrales, de l'épilepsie, d'une altération de la cognition chez les personnes âgées, des troubles neurodégénératifs, des situations de stress physique et physiologique.

8. Forme tautomère énolique de l'acide 3-indolyl-pyruvique selon la revendication 5, destinée à être utilisée dans un traitement de pathologies cardiovasculaires.

9. Forme tautomère énolique de l'acide 3-indolyl-pyruvique selon la revendication 5, destinée à être utilisée dans un traitement thérapeutique pour le bourgeonnement des cellules neuronales.

10. Forme tautomère énolique de l'acide 3-indolyl-pyruvique selon la revendication 5, destinée à être utilisée dans un traitement thérapeutique de tumeurs.

11. Forme tautomère énolique de l'acide 3-indolyl-indolyle-pyruvique selon l'une quelconque des revendications 1 à 4, destinée à être utilisée dans l'industriepharmaceutique sous la forme d'une substance chimiquement pure et stable à la conservation pour la fabrication de médicaments.

12. Procédé de production de la forme tautomère énolique de l'acide 3-indolyl-pyruvique selon l'une quelconque des revendications 1 à 4, qui comprend la réaction de la fonction amino du tryptophane avec un aldéhyde aromatique, en présence d'un agent complexant et d'une base amine accepteur de protons, dans un solvant de réaction et l'hydrolyse du produit de ladite réaction de couplage dans une étape d'hydrolyse acide à chaud à une température de 45 à 65°C, dans lequel : l'aldéhyde aromatique est le 2-pyridine-carboxyaldéhyde seul ou en combinaison avec la triéthylamine,
la base amine non nucléophile accepteur de protons est la triéthylamine,
l'agent complexant est choisi dans le groupe constitué par l'acétate de zinc ou le sulfate de zinc,
et le solvant organique polaire en tant qu'un solvant de réaction est choisi dans le groupe constitué par le tétrahydrofurane, le diméthylformamide, l'acétonitrile, et le méthanol. 1.

13. Procédé selon la revendication 12, dans lequel ledit agent complexant est l'acétate de Zn bihydrate, ou le sulfate de Zn heptahydrate.

14. Procédé selon la revendication 12 ou 13, dans lequel la température de ladite étape d'hydrolyse acide à chaud est 55°C.

15. Procédé selon l'une quelconque des revendications 12, 13 ou 14, dans lequel ledit solvant de réaction n'est pas anhydre.

16. Sel avec un métal alcalin ou alcalino-terreux de la forme tautomère énolique de l'acide 3-indolyl-pyruvique selon l'une quelconque des revendications 1 à 4, sous une forme cristalline pure et stable.

17. Sel selon la revendication 16, dans lequel ledit métal alcalin ou alcalino-terreux est le sodium, le lithium, le potassium, le magnésium ou le calcium.

18. Sel de la forme tautomère énolique de l'acide 3-indolyl-pyruvique selon la revendication 17 destiné à être utilisé en tant qu'un médicament.

19. Sel de la forme tautomère énolique de l'acide 3-indolyl-pyruvique selon la revendication 18, destiné à être utilisé dans le traitement thérapeutique de troubles du système nerveux central.

20. Sel selon la revendication 19, dans lequel lesdits troubles sont l'anxiété, la dépression, les troubles du sommeil, les troubles de l'appétit, les troubles de l'humeur et de l'émotivité, les troubles cérébraux post-traumatiques, l'hypoxie et l'ischémie cérébrales, l'épilepsie, une altération de la cognition chez les personnes âgées, les troubles neurodégénératifs, les situations de stress physique et physiologique.

21. Sel de la forme tautomère énolique de l'acide 3-indolyl-pyruvique selon la revendication 18, destiné à être utilisé dans un traitement thérapeutique de pathologies cardiovasculaires.

22. Sel de la forme tautomère énolique de l'acide 3-indolyl-pyruvique selon la revendication 18, destiné à être utilisé en tant qu'un agent actif pour un traitement thérapeutique d'une déficience de l'activité sérotoninergique ou d'un excès d'activité des acides aminés excitateurs.

23. Sel selon la revendication 18, destiné à être utilisé en tant qu'un agent actif dans un traitement thérapeutique pour le bourgeonnement des cellules neuronales.

24. Sel selon la revendication 18, destiné à être utilisé en tant qu'un agent actif dans le traitement thérapeutique de tumeurs.

25. Procédé de production d'un sel de métal alcalin ou alcalino-terreux selon la revendication 16 ou 17 de la forme tautomère énolique de l'acide 3-indolyl-pyruvique, **caractérisé par** l'étape consistant à neutraliser ledit tautomère énolique de l'acide 3-indolyl-pyruvique avec un métal alcalin ou alcalino-terreux, dans un premier solvant polaire aprotique, conjointement à un deuxième solvant polaire protique en tant qu'un activateur de métal.

26. Procédé selon la revendication 25, dans lequel ledit premier solvant polaire aprotique et ledit deuxième solvant polaire protique sont anhydres.

27. Procédé selon la revendication 25 ou 26, dans lequel ledit solvant polaire aprotique est l'éther diéthylique ou le tétrahydrofurane.

28. Procédé selon l'une quelconque des revendications 25 à 27, dans lequel lesdits métaux neutres sont sous la forme de méthoxydes et d'éthoxydes et ledit solvant protique est l'alcool méthylique ou l'alcool éthylique.

29. Procédé selon l'une quelconque des revendications 25 à 28, dans lequel ledit métal alcalin ou alcalino-terreux est le sodium, le lithium, le potassium, le magnésium ou le calcium.

30. Forme tautomère énolique de l'acide 3-indolyl-pyruvique selon l'une quelconque des revendications 1 à 4, ou un sel alcalin ou alcalino-terreux de celui-ci selon la revendication 16 ou 17, destinée à être utilisée en tant qu'un précurseur de l'acide 3-indolyl-lactique, pour un traitement thérapeutique de patients nécessitant l'administration d'acide 3-indolyl-lactique.

31. Forme tautomère énolique de l'acide 3-indolyl-pyruvique selon l'une quelconque des revendications 1 à 4, ou un sel alcalin ou alcalino-terreux de celui-ci selon la revendication 16 ou 17, destinée à être utilisée en tant qu'un précurseur de l'acide kynurénique, pour un traitement thérapeutique de patients nécessitant 'administration d'acide kynurénique.

32. Composition pharmaceutique comprenant la forme tautomère énolique de l'acide 3-indolyl-pyruvique selon les revendications 1 à 4, ou un sel de celui-ci avec un métal alcalin ou alcalino-terreux selon l'une quelconque des revendications 16 ou 17, ainsi qu'un véhicule ou un excipient pharmaceutiquement compatibles.

33. Composition pharmaceutique selon la revendication 32, dans laquelle ledit véhicule est une solution physiologique.

34. Composition pharmaceutique selon la revendication 32 ou 33, destinée à être utilisée dans un traitement thérapeutique du système nerveux central, des pathologies cardiovasculaires, du bourgeonnement de neurones et des tumeurs.

35. Composition pharmaceutique selon la revendication 32 ou 33, destinée à être utilisée dans le traitement de l'anxiété, de la dépression, des troubles du sommeil, des troubles de l'appétit, des troubles de l'humeur et de l'émotivité, des troubles cérébraux post-traumatiques, de l'hypoxie et de l'ischémie cérébrales, de l'épilepsie, d'une altération de la cognition chez les personnes âgées, des troubles neurodégénératifs, des situations de stress physique et physiologique.

36. Composition pharmaceutique selon la revendication 32 ou 33, sous la forme de gélules, comprimés, suppositoires ou ampoules injectables.
